# EUROPÄISCHE PATENTSCHRIFT

(11) **EP 0 337 205 B1**
(45) Veröffentlichungstag und Bekanntmachung des Hinweises auf die Patenterteilung: **13.01.1993**
(21) Anmeldenummer: 89105652.5
(22) Anmeldetag: 30.03.1989
(51) Int. Cl.: C07C 209/78, C07C 211/50

(54) **Verfahren zur Herstellung von mehrkernigen aromatischen Polyaminen**
Process for the preparation of polynuclear aromatic polyamines
Procédé pour la préparation des polyamines aromatiques polynucléaires

(30) Priorität: 12.04.1988 DE 3812083
(43) Veröffentlichungstag der Anmeldung: 18.10.1989
(73) Patentinhaber: BAYER AG, 51368 Leverkusen (DE)
(72) Erfinder: Knöfel, Hartmut, Dr., D-5068 Odenthal (DE); Brockelt, Michael, D-5090 Leverkusen 3 (DE); Petinaux, Marcel, Dr., D-4150 Krefeld (DE); Uchdorf, Rudolf, D-4150 Krefeld 11 (DE)

(56) Entgegenhaltungen:
- EP-A- 0 288 892
- DE-A- 2 500 573
- DE-A- 2 500 574

## Beschreibung

Die Erfindung betrifft eine verbesserte Ausführungsform des Verfahrens der Herstellung von mehrkernigen aromatischen Polyaminen durch Kondensation von Anilin mit Formaldehyd in Gegenwart von Wasser und sauren Katalysatoren und Aufarbeitung des Reaktionsgemisches durch Extraktion mit einem hydrophoben Lösungsmittel unter Wiederverwendung des in der wäßrigen Phase der Extraktion anfallenden Säurekatalysators.

Es ist bereits bekannt, bei der Herstellung von mehrkernigen aromatischen Polyaminen durch Kondensation von Anilin mit Formaldehyd in Gegenwart von Wasser und sauren Katalysatoren das anfallende wäßrige Reaktionsgemisch durch Extraktion mit einem hydrophoben Lösungsmittel aufzuarbeiten und den bei der Extraktion in der wäßrigen Phase anfallenden Säurekatalysators wiederzuverwenden (vgl. z.B. DE-OS 2 238 920, DE-OS 2 343 658, DE-OS 2 356 828, DE-OS 2 500 573, DE-OS 2 500 574 oder DE-OS 2 528 694).

Der wesentliche Vorteil der Verfahren dieser Vorveröffentlichungen ist darin zu sehen, daß auf eine Neutralisation des Katalysators verzichtet werden kann, da er bei der extraktiven Aufarbeitung des sauren Reaktionsgemischs in der wäßrigen Phase anfällt, die als solche an den Prozeßanfang zurückgeführt und wiederverwendet wird. Außerdem gestatten bestimmte Varianten dieses bekannten Prinzips, wie sie beispielsweise in DE-OS 2 500 574 oder 2 528 694 beschrieben sind, die gezielte Herstellung von Polyamingemischen mit wahlweise erhöhtem oder erniedrigtem Gehalt an 2,4'-Isomeren. Im übrigen entsprechen die Verfahrensprodukte der Vorveröffentlichungen bezüglich ihrer Eignung als Vorprodukt zur Herstellung von Polyisocyanaten den klassischen Polyaminen der Diphenylmethanreihe, die unter Neutralisation des eingesetzten Säurekatalystors hergestellt worden sind. Dies bedeutet, daß das Eigenschaftniveau der aus derartigen Polyisocyanatgemischen der Diphenylmethanreihe hergestellten Polyurethanschaumstoffe in beiden Fällen annähernd das gleiche ist. Es ist aber als ein Nachteil der Verfahren der genannten Vorveröffentlichungen anzusehen, daß zur Aufarbeitung der Verfahrensprodukte durch Extraktion ausschließlich für diesen Zweck beträchtliche Mengen an hydrophobem Lösungsmittel und Anilin eingesetzt werden müssen, was selbstverständlich auf einen beträchtlichen Destillationsaufwand und damit Energieverbrauch bei der destillativen Aufarbeitung der organischen Phase hinausläuft.

Es war die der Erfindung zugrundeliegende Aufgabe, ein neues, verbessertes Verfahren zur Herstellung von mehrkernigen aromatischen Polyaminen aus Anilin und Formaldehyd in Gegenwart von sauren Katalysatoren zur Verfügung zu stellen, welches die Vorteile der bekannten Verfahren des Standes der Technik in sich vereinigt und darüber hinaus die Herstellung von qualitativ verbesserten Produkten bei gleichzeitig reduziertem Destillationsaufwand und damit Energieverbrauch gestattet.

Diese Aufgabe konnte mit dem nachstehend näher beschriebenen erfindungsgemäßen Verfahren gelöst werden. Das erfindungsgemäße Verfahren zeichnet sich insbesondere durch folgende Vorteile aus:
- der eingesetzte Säurekatalysator wird ebenso wie bei den Verfahren des Standes der Technik wiederverwendet und nicht durch Neutralisation vernichtet.
- Die bei der destillativen Aufarbeitung der, die erfindungsgemäßen Verfahrensprodukte enthaltenden, organischen Phase als Destillat anfallenden Gemische, können ohne weitere destillative Auftrennung in ihre Bestandteile als solche, gegebenenfalls nach Zugabe von weiterem Anilin, sowohl als Extraktionsmittel für die wäßrige Phase in der Produktextraktionsstufe als auch am Prozeßanfang wiederverwendet werden.
- Das erfindungsgemäße Verfahren ist bezüglich der Homologenverteilung in den Verfahrensprodukten (Verhältnis von Diaminen zu höherfunktionellen Polyaminen) innerhalb weiter Grenzen variierbar, wobei jedoch stets Produkte mit einem vergleichsweisen geringen Gehalt an ortho-Isomeren anfallen.
- Die aus den erfindungsgemäßen Verfahrensprodukten hergestellten Polyisocyanate liefern überraschenderweise Polyurethanschaumstoffe, die im Vergleich zu entsprechenden Polyurethanschaumstoffen auf Basis der bekannten Polyisocyanatgemische der Diphenylmethanreihe eine deutlich geringere Eigenfarbe aufweisen.
- Im Vergleich zu den genannten "Extraktionsverfahren" des Standes der Technik kann beim erfindungsgemäßen Verfahren die Gesamtmenge des Lösungsmittels wesentlich reduziert werden, so daß die MDA-Konzentration in den anfallenden organischen Phasen beträchtlich erhöht und damit der Destillationsaufwand bei der Aufarbeitung der organischen Phasen entsprechend erniedrigt werden kann.

Gegenstand der Erfindung ist ein Verfahren zur Herstellung von mehrkernigen aromatischen Polyaminen durch Umsetzung von Anilin mit Formaldehyd in Gegenwart von Wasser und sauren Katalysatoren in einer ein- oder zweistufigen Reaktion innerhalb des Temperaturbereichs von 0 bis 180°C, gegebenenfalls unter Vorschaltung einer Aminal-Vorstufe, in welcher in Abwesenheit von Säurekatalysator die Bildung von N,N'-disubstituiertem Aminal stattfindet, welches dann ein- oder mehrstufig in Gegenwart von Säurekatalysator innerhalb des Temperaturbereichs von 0 bis 180°C in das gewünschte Endprodukt überführt wird, Aufarbeitung des resultierenden Reaktionsgemischs durch Extraktion mit einem Anilin-haltigen hydrophoben Lösungsmittel in einer Produktextraktionsstufe (8), destillative Auftrennung der hierbei anfallenden organische Phase in (i) ein aus Anilin-haltigem Lösungsmittel bestehendes Destillat, welches, gegebenenfalls nach Zugabe von Frischanilin erneut bei der Extraktion eingesetzt wird und (ii) einen im wesentlichen aus Verfahrensprodukt bestehenden Destillationsrückstand und Rückführung der bei der Extraktion anfallenden, den Säurekatalysator enthaltenden wäßrigen Phase unter Wiederverwendung des in ihr enthaltenen Katalysators, unter Entfernung des bei der Kondensationsreaktion entstehenden Kondensationswassers und des in das System mit der wäßrigen Lösung des Formaldehyds eingebrachten Wassers in einem der Aminalvorstufe nachgeschalteten und der ersten Reaktionsstufe vorgeschalteten Wasserabscheider und/oder in einem der Extraktionsstufe nachgeschalteten und der ersten Reaktionsstufe vorgeschalteten Verdampfer, dadurch gekennzeichnet, daß man
a) den in Form einer wäßrigen Lösung zum Einsatz gelangenden Formaldehyd in einer Aminal-Vorstufe (3) mit einem Anilin-haltigen hydrophoben Lösungsmittel und/oder in der ersten Reaktionsstufe (5) mit einem Anilin-haltigen hydrophoben Lösungsmittel und der recyclisierten, den Katalysator in Form von Aminsalzen enthaltenden wäßrigen Phase durch Vermischen zur Reaktion bringt,
b) das so erhaltene zweiphasige Reaktionsgemisch nach Beendigung der Reaktion in einem der Extraktionsstufe (8) vorgeschalteten Phasenscheider (7) in eine wäßrige Phase und eine organische Phase auftrennt,
c) die im Phasenscheider (7) anfallende organische Phase in einer der Produktextraktionsstufe (8) nachgeschalteten Nachextraktionsstufe (9) mit in der Produktextraktionsstufe anfallender weitgehend von Reaktionsprodukt befreiter wäßriger Phase extrahiert,
d) die in der Nachextraktionsstufe (9) anfallende mit dem Reaktionsprodukt der im Phasenscheider (7) anfallenden organischen Phase angereicherte wäßrige Phase in den Reaktionsprozess zurückführt,
e) die in der Nachextraktionsstufe (9) anfallende, an Polyarylamin verarmte organische Phase in der Hauptextraktionsstufe (8) als Teil des Extraktionsmittels einsetzt,
f) die im Phasenscheider (7) anfallende wäßrige Phase in der Produktextraktionsstufe (8) mit Anilin und gegebenenfalls Verfahrensprodukt enthaltendem hydrophoben Lösungsmittel extrahiert,
g) die in der Produktextraktionsstufe (8) anfallende organische Phase in der Destillationsstufe (11) in ein aus Anilin-haltigem Lösungsmittel bestehendes Destillat und einen im wesentlichen aus Verfahrensprodukt bestehenden Destillationsrückstand auftrennt und
h) das in der Destillationsstufe (11) anfallende Destillat, gegebenenfalls nach Zugabe von Frischanilin, in zwei Teilströme auftrennt und einen Teilstrom an den Prozeßanfang (3) oder (5) und den anderen Teilstrom zusammen mit der, die Nachextraktionsstufe (9) verlassenden organischen Phase, in der Produktextraktionsstufe (8) als Extraktionsmittel für die wäßrige Phase verwendet.

Ausgangsmaterialien für das erfindungsgemäße Verfahren sind Anilin und Formaldehyd. Der Formaldehyd wird vorzugsweise in Form einer wäßrigen Lösung mit einem Formaldehydgehalt von 20 bis 50 Gew.-% eingesetzt.

Bei den zum Einsatz gelangenden hydrophoben Lösungsmitteln handelt es sich um inerte Lösungsmittel des Siedepunktbereichs 30-250°C, vorzugsweise 80-200°C wie beispielsweise Chlorbenzol, Dichlorbenzole, Benzol, Toluol, Xylol, Dichlorethan, Chloroform oder Tetrachlorkohlenstoff. Vorzugsweise werden Xylole, d.h. technische Xylolgemische und insbesondere o-Xylol als hydrophobes Lösungsmittel verwendet.

Bei dem Säurekatalysator handelt es sich um wasserlösliche Säuren mit einem unter 2,5, vorzugsweise unter 1,5 liegendem pKa-Wert. Beispiele hierfür sind Salzsäure, Bromwaserstoffsäure, Schwefelsäure, Trifluoressigsäure, Methansulfonsäure oder Phosphorsäure. Bevorzugt einzusetzender Katalysator ist Salzsäure. Die genannten Säuren können auch im Gemisch mit sauren oder neutralen Salzen derartiger Säuren, wie z.B. den entsprechenden Ammoniumsalzen oder auch den entsprechenden Alkalisalzen eingesetzt werden; die Mitverwendung derartiger Salze ist jedoch weniger bevorzugt. Die genannten Säuren liegen in dem erfindungsgemäßen Kreislaufsystem in Form der entsprechenden Ammoniumsalze der im wäßrigen Kreislauf befindlichen Basen vor.

Das erfindungsgemäße Verfahren kann sowohl ein- als auch zweistufig und sowohl unter Vorschaltung einer Aminal-Vorstufe als auch ohne eine derartige Aminal-Vorstufe durchgeführt werden, wobei jedoch einschränkend hinzugefügt werden muß, daß bei einstufiger Reaktionsführung die Vorschaltung einer Aminal-Vorstufe auf jeden Fall angezeigt ist.

Unter "einstufiger Reaktionsführung" ist hierbei eine Verfahrensvariante zu verstehen, gemäß welcher das Aminal nach Hinzufügung des Säuekatalysators innerhalb eines kurzen Zeitraums von weniger als 10 Minuten, vorzugsweise von weniger als 5 Minuten auf ein erhöhte Temperatur von 60 bis 180, vorzugsweise 80 bis 150°C erhitzt wird, um dort in das Endprodukt umlagert zu werden, bzw. bei welcher das Aminal direkt mit der auf erhöhtem Temperaturniveau von 60-180°C, vorzugsweise 80 bis 150°C befindlichen und im Kreis geführten wäßrigen Katalystorphase vermischt und das Gemisch dann gegebenenfalls bis zur angestrebten Endtemperatur erhitzt wird.

Unter "zweistufiger Reaktionsführung" ist eine Ausführungsform zu verstehen, gemäß welcher das Aminal nach Zugabe des Säurekatalysators oder das Reaktionsgemisch aus Anilin, Formaldehyd und Säurekatalysator zunächst während eines Zeitraums von 10 bis 90, vorzugweise 15 bis 60 Minuten in einer ersten Reaktionsstufe bei 0 bis 60°C, vorzugsweise 30 bis 60°C und anschließend während eines Zeitraums von 30 bis 180, vorzugsweise 30 bis 120 Minuten in einer zweiten Reaktionsstufe bei 60 bis 180°C, vorzugsweise 60 bis 150°C und insbesondere 100 bis 150°C gehalten wird. Bei dieser bevorzugten Verfahrensvariante einer mehrstufigen, vorzugsweise zweistufigen Reaktionsführung erfolgt in der ersten Reaktionsstufe vor allem eine Umlagerung des Aminals oder (im Falle eines Verzichts auf eine Aminalvorstufe) eine Kondensation der Ausgangsmaterialien zu N-Benzylanilin, welches in der zweiten Reaktionsstufe bei erhöhter Temperatur zum kernsubstituierten Endprodukt umgelagert wird. Gemäß einer besonderen Ausführungsform mit zweistufiger Reaktionsführung - ohne oder vorzugsweise mit Aminalvorstufe - wird die erste Reaktionsstufe nur mit einem Teilstrom der wäßrigen Katalysatorphase, im allgemeinen < 50 %, vorzugsweise < 15 %, durchgeführt. Nach beendeter erster Reaktionsstufe und vor Beendigung der letzten, d.h. im allgemeinen zweiten Reaktionsstufe erfolgt die Vervollständigung der Reaktion in Gegenwart der gesamten Katalysatorphase.

Das Verfahren kann sowohl kontinuierlich als auch diskontinuierlich durchgeführt werden; die angegebenen Zeiträume beziehen sich bei kontinuierlicher Fahrweise auf die mittlere Verweilzeit des Reaktionsgemisches in den einzelnen Stufen. Im Falle der Vorschaltung einer Aminal-Vorstufe beträgt die (mittlere) Verweilzeit der Ausgangsmaterialien in dieser Stufe im allgemeinen 10 bis 60, vorzugsweise 15 bis 60 Minuten. Die Temperatur in der Aminal-Vorstufe liegt im allgemeinen bei 20 bis 100°C, vorzugsweise 20 bis 60°C. Das Verfahren wird in allen Stufen vorzugsweise unter dem Eigendruck des Systems und vorzugsweise in einer Intergasatmosphäre (Stickstoff) durchgeführt.

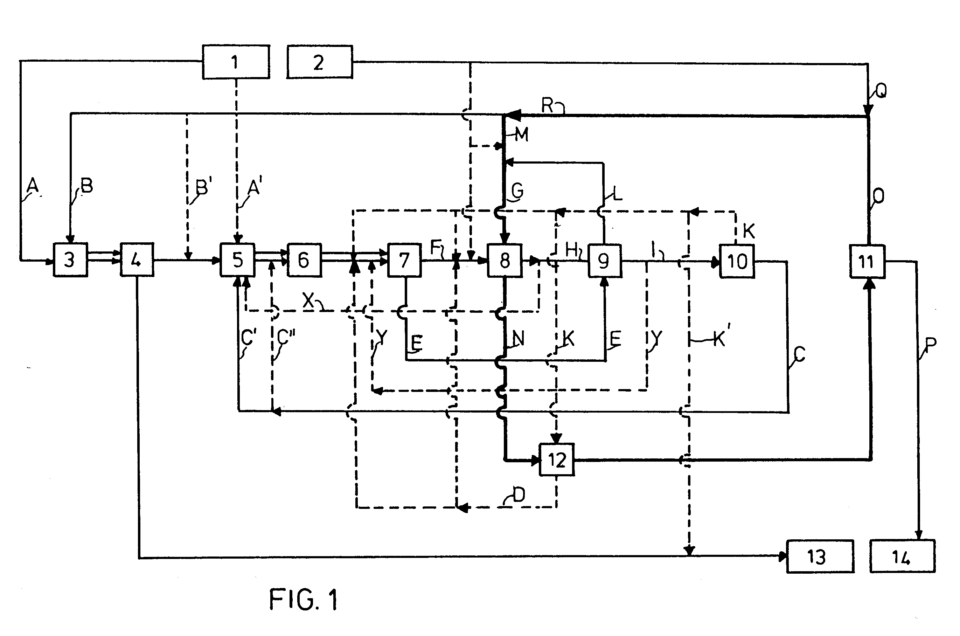

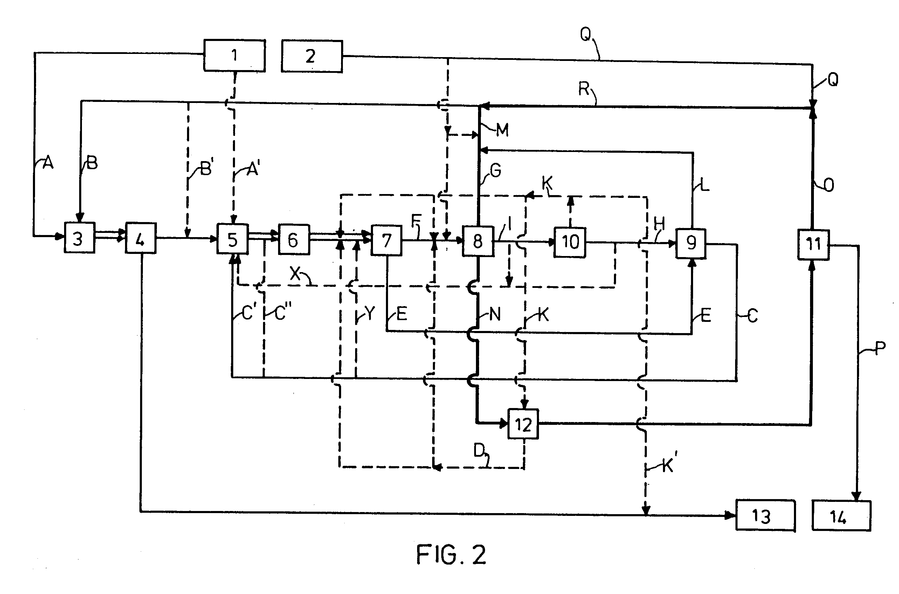

Die in den Figuren 1 und 2 dargestellten Fließdiagramme dienen der weiteren Erläuterung des erfindungsgemäßen Verfahrens. In diesen Abbildungen bedeuten:
(1) einen Tank für wäßrige Formaldehydlösung
(2) einen Tank für Anilin
(3) einen Kondensationsreaktor (Aminal-Vorstufe)
(4) einen Wasserabscheider
(5) die erste Reaktionsstufe
(6) die zweite Reaktionsstufe
(7) einen Phasenscheider
(8) die Produktextraktionsstufe
(9) die Nachextraktionsstufe
(10) einen Wasserverdampfer
(11) die Produktdestillationsstufe
(12) eine Waschstufe
(13) einen Tank für Abwasser
und
(14) einen Tank für Verfahrensprodukt.

Die Bezugszeichen A bis R, sowie X und Y bezeichnen die Mengenströme, auf die nachstehend und in den Beispielen Bezug genommen wird.

Im Falle der genannten einstufigen Reaktionsführung werden die Reaktionsstufe (5) und (6) zu einer einzigen Reaktionsstufe zusammengefaßt. Sowohl die erste als auch die zweite Reaktionsstufe können aus einem einzelnen als auch aus mehreren, in Serie geschalteten Reaktoren bestehen. Zur Aufrechterhaltung der genannten Verweilzeiten haben sich insbesondere in Serie geschaltete Rührkesselkaskaden und oder Kolonnenreaktoren bewährt.

Auch die Extraktionsstufe kann aus einem oder mehreren, in Serie geschalteten Extraktoren bestehen. Hier werden vorzugsweise die üblichen Gegenstromextraktionsvorrichtungen verwendet.

Die Destillationsstufe (11) besteht im einfachsten Fall aus einer Destillsationskolonne, die so ausgelegt ist, daß eine weitgehende Abtrennung von hydrophobem Lösungsmittel und Anilin vom Verfahrensprodukt möglich ist.

Als ein besonderer Vorteil des erfindungsgemäßen Verfahrens ist dabei der Umstand anzusehen, daß die Auftrennung von hydrophobem Lösungsmittel und Anilin nicht erforderlich ist, da der Anilingehalt im Destillat nicht über dem für die Wiederverwendung erforderlichen Wert liegt, der vor der Wiederverwendung je nach Bedarf durch Zugabe von frischem Anilin eingestellt wird, und so der Einsatz energiesparender Mehrstufendestillationstechniken zur Bewältigung der Destillationsaufgabe ermöglich wird.

Das bei der Kondensation entstehende und das mit der wäßrigen Formaldehydlösung in das System eingebrachte Wasser muß an einer geeigneten Stelle zwecks Aufrechterhaltung eines konstanten Wasservolumens aus dem System entfernt werden. Bei Vorschaltung einer Aminalvorstufe (3) geschieht diese Wasserentfernung vorzugsweise im Wasserabscheider (4) bevor das Aminal mit dem Säurekatalysator zusammengebracht wird. In Abwesenheit einer Aminalvorstufe geschieht die Entfernung des Wassers vorzugsweise in einem Wasserverdampfer (10), der der Extraktionsstufe (9) nachgeschaltet oder zwischen der Produktextraktionsstufe (8) und der Nachextraktionsstufe (9) angeordnet ist. Dieser Wasserverdampfer wird vorzugsweise nach dem Prinzip der Entspannungsverdampfung durch Anlegen von Vakuum betrieben.

Grundsätzlich ist es jedoch auch möglich, das Wasser dem System an einer beliebigen anderen Stelle durch Destillation zu entnehmen.

Bei der Durchführung des erfindungsgemäßen Verfahrens sind mehrere Ausführungsformen bzw. Varianten möglich, die nachstehend im Detail beschrieben werden.

Gemäß einer ersten Ausführungsform des erfindungsgemäßen Verfahrens erfolgt die Einspeisung der wäßrigen Formaldehydlösung (A) in die Aminalstufe (3), in welcher die Umsetzung mit einem Gemisch (B) aus Anilin und hydrophobem Lösungsmittel stattfindet. Bei dem Mengenstrom (B) handelt es sich um einen Teil des Destillats (O) aus der Destillationsstufe (11), welchem im allgemeinen eine zusätzliche Menge an Anilin (Q) beigemischt worden ist.

Das Molverhältnis von Anilin zu Formaldehyd liegt im allgemeinen in der Aminalstufe zwischen 1,5:1 und 25:1, vorzugsweise bei 1,8:1 bis 10:1.

Das Gewichtsverhältnis von Anilin zu hydrophobem Lösungsmittel in (B) liegt im allgemeinen zwischen 1:4 und 3:1, vorzugsweise zwischen 1:1 und 2:1.

Die Umsetzung in der Aminalstufe (3) erfolgt innerhalb der obengenannten Temperaturbereiche.

Im Anschluß an (3) erfolgt in einem Scheider (4) die mechanische Abtrennung der wäßrigen Phase, welche von dem Kondensationswasser und dem Formalinwasser gebildet wird und daneben die wasserlöslichen Verunreinigungen des Formaldehyds und Anilins enthält. Die Trennung erfolgt vorzugsweise unterhalb 60°C.

Die verbleibende organische Phase wird in den Reaktor (5) übergeführt und bei Temperaturen unterhalb von 60°C mit dem wäßrigen Mengenstrom (C') zusammengebracht.

Bei dem Mengenstrom (C') handelt es sich bei dieser ersten Ausführungsform des erfindungsgemäßen Verfahrens um die Gesamtmenge der zurückzuführenden Katalysatorphase (C). Der Gehalt an Anilin-Formaldehyd-Kondensaten in dieser Phase (C) liegt im allgemeinen bei 10 bis 40 Gew.-%, vorzugsweise 15 bis 30 Gew.-% bei einem Gesamtgehalt an Arylamin (inklusive Anilin) an dieser Stelle im allgemeinen von 30-70 Gew.-%, vorzugsweise 40-60 Gew.-%, und bei einem Protonierungsgrad von 25 bis 75, vorzugsweise 45 bis 65 %. Unter "Protonierungsgrad" ist hierbei und auch nachstehend der Prozentsatz der Aminstickstoffatome zu verstehen, die in Form von Ammoniumgruppen, d.h. "protoniert" vorliegen.

Das Gewichtsverhältnis zwischen Katalysatorphase (C) und organischer Phase aus (4) liegt im allgemeinen zwischen 1:10 und 100:1, vorzugsweise zwischen 0,5:1 und 3:1.

Bei der ersten, kontinuierlich betriebenen Ausführungsform des erfindungsgemäßen Verfahrens stellt der Reaktor (5) die bereits obengenannte "erste Reaktionsstufe" dar, die unter den obengenannten Bedingungen bezüglich der Temperatur und der Reaktionszeit betrieben wird. Im allgemeinen handelt es sich um eine mehrstufige Rührkesselkaskade oder einen ein- oder mehrstufigen Kolonnenreaktor, in welchem vorzugsweise ein Temperaturprofil von ca. 20°C am Beginn ansteigend bis auf 60°C am Ende durchlaufen wird.

Aus der ersten Reaktionsstufe (5) wird das zweiphasige Reaktionsgemisch in die zweite Reaktionsstufe (6) überführt, die ebenfalls aus einer mehrstufigen Rührkesselkaskade oder einem ein- oder mehrstufigen Kolonnenreaktor besteht. Diese zweite Reaktionsstufe wird ebenfalls unter den bereits obengenannten Bedingungen bezüglich der Reaktionstemperatur und der mittleren Verweilzeit betrieben. Vorzugsweise durchläuft das zweiphasige Reaktionsgemisch in der Reaktionsstufe (6) ein Temperaturprofil beginnend bei 60°C und endend bei einer Temperatur zwischen 95 und 160°C, vorzugsweise zwischen 120 und 140°C. Im Falle dieser bevorzugten Temperaturführung sind in der Reaktionsstufe (6) im allgemeinen Verweilzeiten von bis zu 60 Minuten ausreichend.

Das die zweite Reaktionsstufe (6) verlassende zweiphasige Reaktionsgemisch wird anschließend im Phasenscheider (7), vorzugsweise bei Temperaturen zwischen 80 und 100°C in eine organische Phase (E) und eine wäßrige Phase (F) zerlegt.

Aus der wäßrigen Phase (F) werden in dem vorzugsweise mehrstufig wirkenden Extraktor (8), der vorzugsweise bei Temperaturen von 80 bis 110°C betrieben wird, die Verfahrensprodukte im Austausch gegen Anilin extrahiert und in eine organische Lösung (N) überführt.

Als Extraktionsmittel (G) dient ein Gemisch aus hydrophobem Lösungsmittel und Anilin, das gegebenenfalls geringe Anteile an Anilin-Formaldehyd-Kondensaten enthalten kann. Das Gewichtsverhältnis Anilin (+ Anilin/Formaldehyd-Kondensate) : Lösungsmittel liegt im allgemeinen zwischen 0,5:1 und 3:1, vorzugsweise 1:1 und 2:1.

Das Gewichtsverhältnis Extraktionsmittel (G) : wäßrige Phase (F) liegt im allgemeinen zwischen 0,5:1 und 3:1, vorzugsweise bei 0,7:1 bis 2:1.

Die organische Phase (N) wird, gegebenenfalls nach Durchlaufen der Katalysatorwaschstufe (12), in der gegebenenfalls Katalysatorspuren entfernt werden, in die Destillationsstufe (11) überführt.

In der Destillationsstufe (11) erfolgt die destillative Abtrennung eines Destillationsrückstandes (P), der das Verfahrensprodukt darstellt und in Tank (14) gesammelt wird.

Die Destillationsstufe (11) kann beispielsweise aus einem einstufigen Verdampfer bestehen, der neben dem Destillationsrückstand (P) ein Destillat (O) liefert.

Das Destillat (O) enthält neben Anilin das gesamte hydrophobe Lösungsmittel aus (N) und wird zur Herstellung der Lösungen (B) und (G) verwendet.

Da jedoch der Anilingehalt in (O) stets unter der im Extraktionsmittel (G) erforderlichen Anilinkonzentration liegt, muß dieser Fehlbetrag ergänzt werden. Dies kann beispielsweise durch Vereinigung von (O) mit Frischanilin (Q) aus Tanks (2) erfolgen, wobei der Mengenstrom (R) entsteht. Dieser Mengenstrom (R) wird schließlich in die Mengenströme (B) und (M) aufgeteilt.

Der Mengenstrom (B) wird in die Aminalstufe (3) zurückgeführt, während der Mengenstrom (M) mit dem Mengenstrom (L) zum Extraktionsmittel (G) vereinigt wird.

Es ist jedoch auch möglich, den Mengenstrom (B) in der Zusammensetzung von (O) am Prozeßanfang einzusetzen. In einem solchen Fall wird nur der in (G) eingehende Teilstrom (M) mit einer Teilmenge von (Q) auf die gewünschte Zusammensetzung gebracht und die Restmenge von (Q) an einer geeigneten Stelle, beispielsweise zwischen (6) und (8) dem zweiphasigen System (zwischen (6) und (7)) bzw. der wäßrigen Phase (zwischen (7) und (8)) zugemischt.

Die wäßrige Phase (H) aus der Hauptextraktionsstufe (8) enthält nur noch sehr geringe Anteile von unter 5 Gew.-%, vorzugsweise von unter 2 Gew.-% an Verfahrensprodukten (Anilin/Formaldehyd-Kondensate) und wird in der mehrstufig arbeitenden Nachextraktionsstufe (9), die im allgemeinen bei Temperaturen von 40 bis 110°C betrieben wird, mit dem Mengenstrom (E), d.h. der im Scheider (7) abgetrennten organischen Phase extrahiert, wobei die in (E) enthaltenen Verfahrensprodukte praktisch vollständig gegen Anilin ausgetauscht und in die wäßrige Phase (I) überführt werden, so daß eine organische Phase (L) resultiert, die praktisch frei von Verfahrensprodukten ist.

Die in der Extraktionsstufe (9) resultierende organische Phase (L) ist Teil des Extraktionsmittels (G), so daß sich dieses im allgemeinen aus der Teilmenge (M) des Stroms (R) und der organischen Phase (L) zusammensetzt. Es ist jedoch auch möglich, die Extraktion in (8) in einem mehrstufig wirkenden Extraktor derart durchzuführen, daß Strom (M) zunächst allein in der ersten, bzw. bezogen auf die wäßrige Phase letzten Stufe des Extraktors (8) eingesetzt wird und der Strom (L) in einer späteren bzw. bezogen auf die wäßrige Phase früheren Stufe des Extraktors (8) zugeführt wird.

Die in (9) resultierende wäßrige Phase (I) kann als solche als zurückzuführende Katalysatorlösung (C) verwendet werden.

Vorzugsweise wird jedoch der wäßrigen Lösung (I) im Verdampfer (10) eine Wassermenge (K) entzogen, die im allgemeinen bis zu 80 Gew.-%, vorzugsweise jedoch weniger als 50 Gew.-% des in der wäßrigen Phase in (8) enthaltenden Wassers ausmachen kann. Diese Wassermenge (K) wird dem Reaktionsgemisch zwischen der Reaktionsstufe (6) und der Hauptextraktionsstufe (8) zugeschlagen oder zusätzlich noch dazu benutzt, in der Katalysator-Waschstufe (12) aus der organischen Phase (N) letzte Katalysatorreste herauszuwaschen, um anschließend als Mengenstrom (D) zwischen (6) und (8) dem Reaktionsgemisch zugeschlagen zu werden.

Bei dieser Arbeitsweise (Entfernung von Wasser in (10) und Rückführung) wird die Umlagerung in den Reaktoren (5) und (6) bei einem geringeren Wassergehalt der wäßrigen Phase durchgeführt als die Extraktion in der Extraktionsstufe (8). Dies kann in bestimmten Fällen zu einer Erleichterung der Extraktion in (8) führen.

Zu der beschriebenen 1. Ausführungsform des erfindungsgemäßen Verfahrens sind verschiedene Varianten denkbar.

Eine erste Variante besteht darin, auf die Aminalstufe (3) teilweise oder vollständig zu verzichten. In der Praxis bedeutet dies, daß eine Teilmenge des in der Reaktion eingesetzten Gemischs aus Anilin und hydrophobem Lösungsmittel (B') und eine Teilmenge des eingesetzten wäßrigen Formaldehyds (A') nicht in die Aminalstufe (3) sondern vor bzw. in die erste Reaktionsstufe (5) geleitet werden. Im Extremfall (völliger Verzicht auf eine Aminalvorstufe) kann auch die Gesamtmenge des Gemischs aus Anilin und hydrophobem Lösungsmittel und die Gesamtmenge des wäßrigen Formaldehyds direkt zur ersten Reaktionsstufe (5) geleitet werden. Bei Verzicht auf eine Aminalvorstufe muß jedoch, wie oben ausgeführt, die Reaktion auf jeden Fall zweistufig unter Verwendung der Reaktionsstufen (5) und (6) durchgeführt werden. Bei einer solchen Arbeitsweise wird naturgemäß das eingebrachte und durch die Kondensation entstehende Wasser nur teilweise oder überhaupt nicht über den Phasenscheider (4) entfernt. Die Entfernung dieses Wassers wäre dann beispielsweise durch Abzweigen eines Teilstroms (K') aus dem Destillat aus (10) denkbar, der direkt in den Abwassertank (13) geleitet wird.

Gemäß einer zweiten Variante können die Reaktionsstufen (5) und (6) zu einer einzigen Reaktionsstufe zusammengefaßt werden, die dann unter den obengenannten Bedingungen bezüglich der Reaktionstemperatur und der Reaktionszeit betrieben wird. Im Falle einer einstufigen Reaktionsführung ist jedoch auf jeden Fall eine vorgeschaltete Aminalstufe (3) erforderlich.

Sowohl bei der ersten als auch bei der zweiten Variante werden die Mengenströme im übrigen so bemessen, daß in (5) ein Anilin/Formaldehyd-Molverhältnis von 1,5:1 bis 25:1 vorliegt, wobei das mit dem Katalysatorstrom (C) eingebrachte Arylamin mit in die Bilanz eingeht.

Gemäß einer dritten, mit der ersten Variante kombinierbaren Variante erfolgt in der ersten Reaktionsstufe die Umsetzung in Gegenwart von lediglich einem Teil (C') der zurückgeführten Katalysatorlösung und unter Zugabe der verbleibenden Menge an Katalysatorlösung (C'') zwischen den Reaktionsstufen (5) und (6). Das Gewichtsverhältnis zwischen organischer Phase in (5) und zunächst zugeführter wäßriger Phase (C') z.B. im ersten Rührkessel von (5) liegt zwischen 1:1 und 100:1, vorzugsweise zwischen 3:1 und 30:1.

Gemäß einer vierten, mit den vorgenannten Varianten kombinierbaren Variante wird der wäßrigen, die Hauptextraktionsstufe (8) verlassenden Phase eine Teilmenge (X) entzogen, die bis zu 50 Gew.-% vorzugsweise bis zu 15 Gew.-% der gesamten wäßrigen Phase aus (8) betragen kann, und in die erste Reaktionsstufe zurückgeführt. In einem solchen Falle wird vorzugsweise die Gesamtmenge der zurückgeführten Katalysatorphase (C) als Strom (C'') hinter (5) in das System geleitet. Der Katalysator in (5) besteht dann praktisch ausschließlich aus in (X) vorliegenden Anilinhydrochloriden, während in der Katalysatorphase (C) neben Anilinhydrochloriden auch noch Hydrochloride von Verfahrensprodukten vorliegen.

Gemäß einer fünften Variante, die mit den vorgehenden Varianten kombinierbar ist, wird der wäßrigen, die Nachextraktionsstufe (9) verlassenden Phase (I) ein Teilstrom (Y) entzogen und vor (7) in das Reaktionsgemisch zurückgeführt. Diese Variante ist insbesondere dann von Interesse, wenn das Verhältnis von organischer Phase zu wäßriger Phase in der Nachextraktionsstufe (9) erniedrigt werden soll, um so gegebenenfalls die Nachextraktionsstufe (9) unter optimalen Bedingungen betreiben zu können, d.h. bei einem Phasengewichtsverhältnis von (E) zu (H) von unter 1,5:1, vorzugsweise von unter 1:1.

Die zweite Ausführungsform des erfindungsgemäßen Verfahrens unterscheidet sich von der ersten Ausführungsform dadurch, daß die Verdampferstufe (10) vor der Nachextraktionsstufe (9) angeordnet ist.

Diese zweite Ausführungsform ist ebenfalls in den obengenannten Varianten zur ersten Ausführungsform durchführbar, wobei hier jedoch bei der vierten Variante der Strom (X) auch hinter der Verdampferstufe (10) und vor der Nachextraktionsstufe (9) entnommen werden kann, und wobei bei der fünften Variante der Strom (Y) einen Teil der zurückgeführten wäßrigen Katalysatorlösung (C) darstellt.

Eine Umkehr der Reihenfolge der Stufen (9) und (10), wie sie in Fig. 2 dargestellt ist, hat eine Erhöhung des Verhältnisses von organischer Phase (E) zu wäßriger Phase (H) bei gleicher Säuremenge in der Phase (H) zur Folge, wodurch ebenfalls eine Optimierung des Wirkungsgrades der Nachextraktionsstufe (9) möglich ist.

Der Hauptzweck der Nachextraktionsstufe (9) besteht in der praktisch restlosen Entfernung von Anilin/Formaldehyd-Kondensaten aus der organischen Phase (E), was im allgemeinen dann erreicht wird, wenn das Phasen-Gewichtsverhältnis von (E) zu (H) unter 1,5:1, vorzugsweise unter 1:1 liegt.

In den nachfolgenden Beispielen beziehen sich alle Prozentangaben auf Gewichtsprozente. Der Begriff "Polyarylamin" steht jeweils als Sammelbegriff für alle in den jeweiligen Mengenströmen als Gemisch vorliegenden Polyamine der Diphenylmethanreihe.

### Beispiel 1 (Figur 1)

In einem aus zwei hintereinandergeschalteten Rührkesseln bestehenden Reaktor (3) wird 30 %ige wäßrige Formalinlösung (Mengenstrom A) mit einem Anilin-Xylolgemisch (Mengenstrom B) bei 40°C zur Reaktion gebracht.
- (A): 0,375 kg/h Formaldehyd
0,875 kg/h Wasser
- (B): 2,327 kg/h Anilin
1,940 kg/h ortho-Xylol
In dem anschließenden Scheider (4) wird die untere, wäßrige Phase als Abwasser abgetrennt und in dem Abwassertank (13) gesammelt.

Die obere, organische Phase wird in einen zweiten, aus drei Rührkesseln bestehenden Reaktor (5) übergeführt und dort mit dem Mengenstrom (C) vermischt.
- (C): 0,881 kg/h Polyarylamin
0,563 kg/h Anilin
0,328 kg/h Chlorwasserstoff
1,975 kg/h Wasser
Die gemessenen und geregelten Temperaturen in den drei Kesseln des Reaktors (5) betragen 30°C, 40°C und 60°C.

In einem weiteren, ebenfalls aus drei Rührkesseln bestehenden Reaktor (6) werden die Temperaturen 100°C, 135°C und 140°C durchlaufen, die durch Aufheizen unter dem sich einstellenden Eigendruck des Systems eingestellt werden.

Nach dem Abkühlen des Reaktorgemisches auf 95°C und Entspannen auf Normaldruck und nach Zugabe des HCl-Waschwassers aus der Waschstufe (12) (Mengenstrom D) werden im Phasenscheider (7) die organische (Mengenstrom E) und die wäßrige Phase (Mengenstrom F) voneinander getrennt.
- (E): 0,870 kg/h Polyarylamin
0,703 kg/h Anilin
1,940 kg/h ortho-Xylol
- (F): 2,130 kg/h Polyarylamin
0,218 kg/h Anilin
0,328 kg/h Chlorwasserstoffsäure
2,881 kg/h Wasser
Die wäßrige Phase (F) wird anschließend in der Extraktionskolonne (8) im Gegenstrom mit einem Anilin-Xylolgemisch (Mengenstrom G) kontinuierlich extrahiert
- (G): 0,038 kg/h Polyarylamin
4,159 kg/h Anilin
4,143 kg/h ortho-Xylol
und geht dabei über in die an Polyarylamin verarmte wäßrige Phase (H).
- (H): 0,049 kg/h Polyarylamin
1,370 kg/h Anilin
0,328 kg/h Chlorwasserstoffsäure
2,881 kg/h Wasser
welche in einer weiteren Extraktionskolonne (9) zur Gegenstromextraktion der in (7) abgetrennten organischen Phase (E) eingesetzt wird.

Die in (9) resultierende, gegenüber der eingesetzten wäßrigen Phase (H) an Polyarylamin angereicherte wäßrige Phase (Mengenstrom I)
- (I): 0,881 kg/h Polyarylamin
0,563 kg/h Anilin
0,328 kg/h Chlorwasserstoffsäure
2,881 kg/h Wasser
wird in der Destillationsstufe (10) aufkonzentriert unter Entnahme des Destillats als Mengenstrom (K) und anschließend als Mengenstrom (C) in Reaktor (5) zurückgeführt.
- (K): 0,906 kg/h Wasser
Die in der Extraktionsstufe (9) anfallende an Polyarylamin gegenüber (E) verarmte organische Phase wird als Extraktionsmittel in der Produktextraktionsstufe (8) verwendet und bildet als Mengenstrom (L) zusammen mit Anilin-Xylolgemisch (M) die Gesamtmenge des in der Produkextraktionsstufe (8) eingesetzten Extraktionsmittels (Mengenstrom G)
- (L): 0,038 kg/h Polyarylamin
1,510 kg/h Anilin
1,940 kg/h ortho-Xylol
- (M): 2,649 kg/h Anilin
2,203 kg/h ortho-Xylol
Die in der Produktextraktionsstufe (8) anfallende, das Reaktionsprodukt enthaltende organische Phase (Mengenstrom N) wird in einer weiteren 3 bis 5 stufig wirkenden Extraktionskolonne (Waschsstufe) (12) mit dem im wesentlichen aus Wasser bestehenden Destillat der Destillationsstufe (10) (Mengenstrom K) extrahiert.
- (N): 2,119 kg/h Polyarylamin
3,007 kg/h Anilin
4,143 kg/h ortho-Xylol
- (K): 0,906 kg/h Wasser
In der Waschstufe (12) wird der HCl-Gehalt des Mengenstroms (N), der bei ca. 0,2-0,3 Gew.-% liegt, unter den angegebenen Bedingungen reduziert auf <0,01 Gew.-%. Das HCl-haltige Waschwasser wird als Mengenstrom (D) in das Reaktionsgemisch zwischen (6) und (7) recyclisiert.

Die die Waschkolonne (12) verlassende organische Phase wird in einer Destillationsstufe (11) aufgetrennt in ein Destillat (Mengenstrom O) und einen Destillationsrückstand (Mengenstrom P).
- (O): 3,007 kg/h Anilin
4,143 kg/h ortho-Xylol
- (P): 2,119 kg/h Polyarylamin
Nach Zugabe von frischem Anilin (Mengenstrom Q) aus dem Vorratstank (2) zu dem Mengenstrom (O) wird das so hergestellte Anilin-Xylolgemisch (Megenstrom R) aufgeteilt und als Mengenströme (B) und (M) eingesetzt.

Der Destillationsrückstand (Megenstrom P) der Destillationsstufe (11) hat folgende Zusammensetzung:
0,2 % 2,2'-Diaminodiphenylmethan
4,3 % 2,4'-Diaminodiphenylmethan
46,3 % 4,4'-Diaminodiphenylmethan
0,2 % N-Methyl-substituierte Diaminodiphenylmethane
22,2 % Triamine
11,1 % Tetramine
15,6 % höher als tetrafunktionelle Polyamine

### Beispiel 2 (Figur 1)

In einem aus zwei hintereinandergeschalteten Rührkesseln bestehenden Reaktor (3) wird 30 %ige wäßrige Formalinlösung (Mengenstrom A) aus Vorratstank (1) mit einem Anilin-Xylolgemisch (Mengenstrom B) bei 40°C zur Reaktion gebracht.
- (A): 0,500 kg/h Formaldehyd
1,166 kg/h Wasser
- (B): 3,103 kg/h Anilin
2,586 kg/h ortho-Xylol
In den anschließenden Scheider (4) wird die untere, wäßrige Phase als Abwasser abgetrennt und in dem Abwassertank (13) gesammelt.

Die obere, organische Phase wird in einen zweiten, aus drei Rührkesseln bestehenden Reaktor (5) übergeführt und dort mit dem Mengenstrom (C) vermischt.
- (C): 0,805 kg/h Polyarylamin
1,771 kg/h Anilin
0,586 kg/h Chlorwasserstoff
3,530 kg/h Wasser
Die gemessenen und geregelten Temperaturen in den drei Kesseln des Reaktors (5) betragen 35°C, 50°C und 60°C.

In einem weiteren, ebenfalls aus drei Rührkesseln bestehenden Reaktor (6) werden die Temperaturen 100°C, 135°C und 140°C durchlaufen, die durch Aufheizen unter dem sich einstellenden Eigendruck des Systems eingestellt werden.

Nach dem Abkühlen des Reaktorgemisches auf 95°C und Entspannen auf Normaldruck und nach Zugabe des HCl-Waschwassers aus der Waschstufe (12) (Mengenstrom D) werden im Phasenscheider (7) die organische (Mengenstrom E) und die wäßrige Phase (Mengenstrom F) voneinander getrennt.
- (E): 0,734 kg/h Polyarylamin
1,460 kg/h Anilin
2,586 kg/h ortho-Xylol
- (F): 2,897 kg/h Polyarylamin
0,779 kg/h Anilin
0,586 kg/h Chlorwasserstoffsäure
5,148 kg/h Wasser
Die wäßrige Phase (F) wird anschließend in der Extraktionskolonne (8) im Gegenstrom mit einem Anilin-Xylolgemisch (Mengenstrom G) kontinuierlich extrahiert
- (G): 0,048 kg/h Polyarylamin
7,893 kg/h Anilin
7,215 kg/h ortho-Xylol
und geht dabei über in die an Polyarylamin verarmte wäßrige Phase (H).
- (H): 0,110 kg/h Polyarylamin
2,650 kg/h Anilin
0,586 kg/h Chlorwasserstoffsäure
5,148 kg/h Wasser
welche in einer weiteren Extraktionskolonne (9) zur Gegenstromextraktion der in (7) abgetrennten organischen Phase (E) eingesetzt wird.

Die in (9) resultierende, gegenüber der eingesetzten wäßrigen Phase (H) an Polyarylamin ansgereicherte wäßrige Phase (Mengenstrom I)
- (I): 0,805 kg/h Polyarylamin
1,771 kg/h Anilin
0,586 kg/h Chlorwasserstoffsäure
5,148 kg/h Wasser
wird in der Destillationsstufe (10) aufkonzentriert unter Entnahme des Destillats als Mengenstrom (K) und anschließend als Mengenstrom (C) in Reaktor (5) zurückgeführt.
- (K): 1,618 kg/h Wasser
Die in der Extraktionsstufe (9) anfallende an Polyarylamin gegenüber (E) verarmte organische Phase wird als Extraktionsmittel in der Produktextraktionsstufe (8) verwendet und bildet als Mengenstrom (L) zusammen mit Anilin-Xylolgemisch (M) die Gesamtmenge des in der Produkextraktionsstufe (8) eingesetzten Extraktionsmittels (Mengenstrom G)
- (L): 0,048 kg/h Polyarylamin
2,339 kg/h Anilin
2,586 kg/h ortho-Xylol
- (M): 5,554 kg/h Anilin
4,629 kg/h ortho-Xylol
Die in der Produktextraktionsstufe (8) anfallende, das Reaktionsprodukt enthaltende organische Phase (Mengenstrom N) wird in einer weiteren 3 bis 5 stufig wirkenden Extraktionskolonne (Waschstufe) (12) mit dem im wesentlichen aus Wasser bestehenden Destillat der Destillationsstufe (10) (Mengenstrom K) extrahiert.
- (N): 2,835 kg/h Polyarylamin
6,022 kg/h Anilin
7,215 kg/h ortho-Xylol
- (K): 1,618 kg/h Wasser
In der Waschstufe (12) wird der HCl-Gehalt des Mengenstroms (N), der bei ca. 0,2-0,3 Gew.-% liegt, unter den angegebenen Bedingungen reduziert auf <0,01 Gew.-%. Das HCl-haltige Waschwasser wird als Mengenstrom (D) in das Reaktionsgemisch recyclisiert.

Die die Waschstufe (12) verlassende organische Phase wird in einer Destillationsstufe (11) aufgetrennt in ein Destillat (Mengenstrom O) und einen Destillationsrückstand (Mengenstrom P).
- (O): 6,027 kg/h Anilin
7,215 kg/h ortho-Xylol
- (P): 2,835 kg/h Polyarylamin
Nach Zugabe von frischem Anilin (Mengenstrom Q) aus dem Vorratstank (2) zu dem Mengenstrom (O) wird das so hergestellte Anilin-Xylolgemisch (Mengenstrom R) aufgeteilt und als Mengenströme (B) und (M) eingesetzt.

Der Destillationsrückstand (Mengenstrom P) der Destillationsstufe (11) hat folgende Zusammensetzung:
0,4 % 2,2'-Diaminodiphenylmethan
5,9 % 2,4'-Diaminodiphenylmethan
58,7 % 4,4'-Diaminodiphenylmethan
0,2 % N-Methyl-substituierte Diaminodiphenylmethane
20,5 % Triamine
7,7 % Tetramine
6,6 % höher als tetrafunktionelle Polyamine

### Beispiel 3 (Figur 1)

In einem aus zwei hintereinandergeschalteten Rührkesseln bestehenden Reaktor (3) wird 30 %ige wäßrige Formalinlösung (Mengenstrom A) aus Vorratstank (1) mit einem Anilin-Xylolgemisch (Megenstrom B) bei 40°C zur Reaktion gebracht.
- (A): 0,500 kg/h Formaldehyd
1,166 kg/h Wasser
- (B): 3,103 kg/h Anilin
2,586 kg/h ortho-Xylol
In dem anschließenden Scheider (4) wird die untere, wäßrige Phase als Abwasser abgetrennt und in dem Abwassertank (13) gesammelt.

Die obere, organische Phase wird in einen zweiten, aus drei Rührkesseln bestehenden Reaktor (5) übergeführt und dort mit dem Mengenstrom (C) vermischt.
- (C): 0,924 kg/h Polyarylamin
1,000 kg/h Anilin
0,437 kg/h Chlorwasserstoff
2,634 kg/h Wasser
Die gemessenen und geregelten Temperaturen in den drei Kesseln des Reaktors (5) betragen 35°C, 50°C und 60°C.

In einem weiteren, ebenfalls aus drei Rührkesseln bestehenden Reaktor (6) werden die Temperaturen 100°C, 135°C und 140°C durchlaufen, die durch Aufheizen unter dem sich einstellenden Eigendruck des Systems eingestellt werden.

Nach dem Abkühlen des Reaktorgemisches auf 95°C und Entspannen auf Normaldruck werden im Phasenscheider (7) die organische Phase (Mengenstrom E) und die wäßrige Phase (Mengenstrom F) voneinander getrennt.
- (E): 0,910 kg/h Polyarylamin
1,187 kg/h Anilin
2,586 kg/h ortho-Xylol
- (F): 2,844 kg/h Polyarylamin
0,286 kg/h Anilin
0,437 kg/h Chlorwasserstoffsäure
2,634 kg/h Wasser
Die wäßrige Phase (F) wird nach Zugabe des HCl-Waschwassers aus der Extraktionsstufe (12) (Mengenstrom D) anschließend in der Extraktionskolonne (8) im Gegenstrom mit einem Anilin-Xylolgemisch (Mengenstrom G) kontinuierlich extrahiert
- (G): 0,048 kg/h Polyarylamin
5,592 kg/h Anilin
5,523 kg/h ortho-Xylol
und geht dabei über in die an Polyarylamin verarmte wäßrige Phase (H).
- (H): 0,062 kg/h Polyarylamin
1,823 kg/h Anilin
0,437 kg/h Chlorwasserstoffsäure
3,841 kg/h Wasser
welche in einer weiteren Extraktionskolonne (9) zur Gegenstromextraktion der in (7) abgetrennten organischen Phase (E) eingesetzt wird.

Die in (9) resultierende, gegenüber der eingesetzten wäßrigen Phase (H) an Polyarylamin angereicherte wäßrige Phase (Mengenstrom I)
- (I): 0,924 kg/h Polyarylamin
1,000 kg/h Anilin
0,437 kg/h Chlorwasserstoffsäure
3,841 kg/h Wasser
wird in der Destillationsstufe (10) aufkonzentriert unter Entnahme des Destillats als Mengenstrom (K) und anschließend als Mengenstrom (C) in Reaktor (5) zurückgeführt.
- (K): 1,207 kg/h Wasser
Die in der Extraktionsstufe (9) anfallende an Polyarylamin gegenüber (E) verarmte organische Phase wird als Extraktionsmittel in der Produktextraktionsstufe (8) verwendet und bildet als Mengenstrom (L) zusammen mit Anilin-Xylolgemisch (M) die Gesamtmenge des in der Produkextraktionsstufe (8) eingesetzten Extraktionsmittels (Mengenstrom G)
- (L): 0,048 kg/h Polyarylamin
2,060 kg/h Anilin
2,586 kg/h ortho-Xylol

- (M): 3,532 kg/h Anilin
2,037 kg/h ortho-Xylol
Die in der Produktextraktionsstufe (8) anfallende, das Reaktionsprodukt enthaltende organische Phase (Mengenstrom N) wird in einer weiteren 3 bis 5 stufig wirkenden Extraktionskolonne (12) mit dem im wesentlichen aus Wasser bestehenden Destillat der Destillationsstufe (10) (Mengenstrom K) extrahiert.
- (N): 2,830 kg/h Polyarylamin
4,005 kg/h Anilin
5,523 kg/h ortho-Xylol
- (K): 1,207 kg/h Wasser
In der Waschstufe (12) wird der HCl-Gehalt des Mengenstroms (N), der bei ca. 0,2-0,3 Gew.-% liegt, unter den angegebenen Bedingungen reduziert auf <0,01 Gew.-%. Das HCl-haltige Waschwasser wird als Mengenstrom (D) in das Reaktionsgemisch recyclisiert.

Die die Waschkolonne (12) verlassende organische Phase wird in einer Destillationsstufe (11) aufgetrennt in ein Destillat (Mengenstrom O) und einen Destillationsrückstand (Mengenstrom P).
- (O): 4,005 kg/h Anilin
5,523 kg/h ortho-Xylol
- (P): 2,830 kg/h Polyarylamin
Nach Zugabe von frischem Anilin (Mengenstrom Q) aus dem Vorratstank (2) zu dem Mengenstrom (O) wird das so hergestellte Anilin-Xylolgemisch (Mengenstrom R) aufgeteilt und als Mengenströme (B) und (M) eingesetzt.

Der Destillationsrückstand (Mengenstrom P) der Destillationsstufe (11) hat folgende Zusammensetzung:
0,3 % 2,2'-Diaminodiphenylmethan
4,4 % 2,4'-Diaminodiphenylmethan
50,5 % 4,4'-Diaminodiphenylmethan
0,2 % N-Methyl-substituierte Diaminodiphenylmethane
20,7 % Triamine
10,1 % Tetramine
13,8 % höher als tetrafunktionelle Polyamine

## Patentansprüche

1. Verfahren zur Herstellung von mehrkernigen aromatischen Polyaminen durch Umsetzung von Anilin mit Formaldehyd in Gegenwart von Wasser und sauren Katalysatoren in einer ein- oder zweistufigen Reaktion innerhalb des Temperaturbereichs von 0 bis 180°C, gegebenenfalls unter Vorschaltung einer Aminal-Vorstufe, in welcher in Abwesenheit von Säurekatalysator die Bildung von N,N'-disubstituiertem Aminal stattfindet, welches dann ein- oder mehrstufig in Gegenwart von Säurekatalysator innerhalb des Temperaturbereichs von 0 bis 180°C in das gewünschte Endprodukt überführt wird, Aufarbeitung des resultierenden Reaktionsgemischs durch Extraktion mit einem Anilin-haltigen hydrophoben Lösungsmittel in einer Produktextraktionsstufe (8), destillative Auftrennung der hierbei anfallenden organischen Phase in (i) ein aus Anilin- haltigem Lösungsmittel bestehendes Destillat, welches, gegebenenfalls nach Zugabe von Frischanilin erneut bei der Extraktion eingesetzt wird und (ii) einen im wesentlichen aus Verfahrensprodukt bestehenden Destillationsrückstand und Rückführung der bei der Extraktion anfallenden, der Säurekatalysator enthaltenden wäßrigen Phase unter Wiederverwendung des in ihr enthaltenen Katalysators, unter Entfernung des bei der Kondensationsreaktion entstehenden Kondensationswassers und des in das System mit der wäßrigen Lösung des Formaldehyds eingebrachten Wassers in einem der Aminalvorstufe nachgeschalteten und der ersten Reaktionsstufe vorgeschalteten Wasserabscheider und/oder in einem der Extraktionsstufe nachgeschalteten und der ersten Reaktionsstufe vorgeschalteten Verdampfer, dadurch gekennzeichnet, daß man
a) den in form einer wäßrigen Lösung zum Einsatz gelangenden Formaldehyd in einer Aminal-Vorstufe (3) mit einem Anilin-haltigen hydrophoben Lösungsmittel und/oder in der ersten Reaktionsstufe (5) mit einem Anilin-haltigen hydrophoben Lösungsmittel und der recyclisierten, den Katalysator in Form von Aminsalzen enthaltenden wäßrigen Phase durch Vermischen zur Reaktion bringt,
b) das so erhaltene zweiphasige Reaktionsgemisch nach Beendigung der Reaktion in einem der Extraktionsstufe (8) vorgeschalteten Phasenscheider (7) in eine wäßrige Phase und eine organische Phase auftrennt,
c) die im Phasenscheider (7) anfallende organische Phase in einer der Produktextraktionsstufe (8) nachgeschalteten Nachextraktionsstufe (9) mit in der Produktextraktionsstufe anfallender weitgehend von Reaktionsprodukt befreiter wäßriger Phase extrahiert,
d) die in der Nachextraktionsstufe (9) anfallende mit dem Reaktionsprodukt der im Phasenscheider (7) anfallenden organischen Phase angereicherte wäßrige Phase in den Reaktionsprozess zurückführt,
e) die in der Nachextraktionsstufe (9) anfallende, an Polyarylamin verarmte organische Phase in der Hauptextraktionsstufe (8) als Teil des Extraktionsmittels einsetzt,
f) die im Phasenscheider (7) anfallende wäßrige Phase in der Produktextraktionsstufe (8) mit Anilin und gegebenenfalls Verfahrensprodukt enthaltendem hydrophoben Lösungsmittel extrahiert,
g) die in der Produktextraktionsstufe (8) anfallende organische Phase in der Destillationsstufe (11) in ein aus Anilin-haltigem Lösungsmittel bestehendes Destillat und einen im wesentlichen aus Verfahrensprodukt bestehenden Destillationsrückstand auftrennt und
h) das in der Destillationsstufe (11) anfallende Destillat, gegebenenfalls nach Zugabe von Frischanilin, in zwei Teilströme auftrennt und einen Teilstrom an den Prozeßanfang (3) oder (5) und den anderen Teilstrom zusammen mit der, die Nachextraktionsstufe (9) verlassenden organischen Phase, in der Produktextraktionsstufe (8) als Extraktionsmittel für die wäßrige Phase verwendet.

2. Verfahren gemäß Anspruch 1, dadurch gekennzeichnet, daß man der die Produktextraktionsstufe (8) verlassenden wäßrigen Phase vor ihrer Wiederverwendung in einem Wasserverdampfer (10) destillativ eine Wassermenge entzieht, die bis zu 80 Gew.-% der in der wäßrigen Phase aus (8) vorliegenden Wassermenge beträgt, und, gegebenenfalls nach Durchlaufen einer Waschstufe (12) in der aus der organischen Phase aus (8) Katalysatorreste entfernt werden an einer geeigneten Stelle hinter der letzten Reaktionsstufe (6) und vor der Produktextraktionsstufe (8) in den Kreislauf zurückführt.

3. Verfahren gemäß Anspruch 1 und 2, dadurch gekennzeichnet, daß man die, den wiederzuverwendenden Katalysator enthaltende, wäßrige Phase vor ihrer Wiederverwendung in zwei Teilströme auftrennt und den einer Teilstrom in die erste Reaktionsstufe (5) und den anderen Teilstrom nach beendeter erster und vor beeendeter letzer Reaktionsstufe (6) dem Reaktionsgemisch zusetzt.

## Claims

1. A process for the production of polynuclear aromatic polyamines by reaction of aniline with formaldehyde in the presence of water and acidic catalysts in one or two stages at a temperature in the range from 0 to 180°C, optionally preceded by an aminal preliminary stage in which N,N'-disubstituted aminal is formed in the absence of acid catalyst and is then converted into the desired end product in one or more stages in the presence of acid catalyst at a temperature in the range from 0 to 180°C, working up of the resulting reaction mixture by extraction with an aniline-containing hydrophobic solvent in a product extraction stage (8), separation of the organic phase accumulating by distillation into (i) a distillate consisting of aniline-containing solvent which is reused in the extraction stage, optionally after addition of fresh aniline, and (ii) a distillation residue consisting essentially of end product and recycling of the aqueous phase containing the acid catalyst which accumulates during extraction, the catalyst present in this aqueous phase being reused and the water of condensation formed during the condensation reaction and the water introduced into the system with the aqueous solution of the formaldehyde being removed in a water separator following the aminal preliminary stage and preceding the first stage of the reaction and/or in an evaporator following the extraction stage and preceding the first stage of the reaction, characterized in that
a) the formaldehyde used in the form of an aqueous solution is reacted by mixing in an aminal preliminary stage (3) with an aniline-containing hydrophobic solvent and/or in the first stage (5) of the reaction with an aniline-containing hydrophobic solvent and the recycled aqueous phase containing the catalyst in the form of amine salts,
b) on completion of the reaction, the two-phase reaction mixture thus obtained is separated into an aqueous phase and an organic phase in a phase separator (7) preceding the extraction stage (8),
c) the organic phase accumulating in the phase separator (7) is extracted in a re-extraction stage (9) following the product extraction stage (8) with aqueous phase substantially freed from reaction product which accumulates in the product extraction stage
d) the aqueous phase accumulating in the re-extraction stage (9) and enriched with the reaction product of the organic phase accumulating in the phase separator (7) is returned to the reaction process,
e) the polyarylamine-depleted organic phase accumulating in the re-extraction stage (9) is used as part of the extractant in the main extraction stage (8),
f) the aqueous phase accumulating in the phase separator (7) is extracted in the product extraction stage (8) with hydrophobic solvent containing aniline and, optionally, end product,
g) the organic phase accumulating in the product extraction stage (8) is separated in the distillation stage (11) into a distillate consisting of aniline-containing solvent and a distillation residue consisting essentially of end product and,
h) optionally after addition of fresh aniline, distillate accumulating in the distillation stage (11) is separated into two component streams of which one is used at the beginning (3) or (5) of the process while the other is used together with the organic phase leaving the re-extraction stage (9) as extractant for the aqueous phase in the product extraction stage (8).

2. A process as claimed in claim 1, characterized in that a quantity of water of up to 80% by weight of the quantity of water present in the aqueous phase from (8) is removed by distillation from the aqueous phase leaving the product extraction stage (8) before it is reused in a water evaporator (10) and, optionally after passing through a washing stage (12) in which catalyst residues are removed from the organic phase from (8), is returned to the circuit at a suitable point after the last reaction stage (6) and before the product extraction stage (8)

3. A process as claimed in claims 1 and 2, characterized in that, before it is reused, the aqueous phase containing the catalyst to be reused is divided into two streams of which one is introduced into the first reaction stage (5) and the other is added to the reaction mixture before the last reaction stage (6) is complete.

## Revendications

1. Procédé de préparation de polyamines aromatiques polycycliques par réaction de l'aniline avec le formaldéhyde en présence d'eau et de catalyseurs acides dans une réaction à un ou deux stades opératoires à des températures dans l'intervalle de 0 à 180°C, éventuellement avec un stade opératoire préalable de formation d'un aminal dans lequel, en l'absence d'un catalyseur acide, il y a formation d'un aminal N,N'-disubstitué qui est ensuite converti en le produit final recherché en un ou plusieurs stades opératoires en présence d'un catalyseur acide à des températures dans l'intervalle de 0 à 180°C, traitement du mélange de réaction obtenu par extraction à l'aide d'un solvant hydrophobe contenant de l'aniline dans un stade opératoire d'extraction du produit (8), séparation par distillation de la phase organique ainsi obtenue en (i) un distillat consistant en solvant contenant de l'aniline qui, le cas échéant après addition d'aniline fraîche, est réutilisé pour l'extraction et (ii) un résidu de distillation consistant essentiellement en le produit recherché, et recyclage de la phase aqueuse obtenue à l'extraction, contenant le catalyseur acide, avec réutilisation du catalyseur qu'elle contient, et élimination de l'eau de condensation formée à la réaction de condensation et de l'eau introduite dans le système par la solution aqueuse de formaldéhyde dans un séparateur d'eau disposé après l'opération préalable de formation d'un aminal et avant le premier stade de réaction et/ou dans un évaporateur disposé après l'opération d'extraction et avant le premier stade de réaction, caractérisé en ce que
a) on fait réagir le formaldéhyde mis en oeuvre à l'état de solution aqueuse dans un stade préalable de formation d'un aminal (3) par mélange avec un solvant hydrophobe contenant de l'aniline et/ou au premier stade de réaction (5) par mélange avec un solvant hydrophobe contenant de l'aniline et la phase aqueuse recyclée contenant le catalyseur à l'état de sels d'amines,
b) on sépare le mélange de réaction à deux phases ainsi obtenu, lorsque la réaction est terminée, dans un séparateur de phases (7) disposé avant l'opération d'extraction (8), en une phase aqueuse et une phase organique,
c) on extrait la phase organique obtenue au séparateur de phases (7) dans un stade d'extraction supplémentaire (9) disposé après l'opération d'extraction du produit (8) par la phase aqueuse obtenue à l'opération d'extraction du produit, pratiquement entièrement débarrassée du produit de réaction,
d) on recycle à la réaction la phase aqueuse obtenue à l'opération d'extraction supplémentaire (9), enrichie en le produit de réaction de la phase organique obtenue au séparateur de phases (7),
e) on utilise la phase organique obtenue à l'opération d'extraction supplémentaire (9), appauvrie en polyarylamine, à l'opération d'extraction principale (8), en tant que partie de l'agent d'extraction,
f) on extrait la phase aqueuse obtenue au séparateur de phases (7) au stade d'extraction du produit (8) par un solvant hydrophobe contenant de l'aniline et le cas échéant le produit recherché,
g) on sépare la phase organique obtenue à l'opération d'extraction du produit (8), au stade distillation (11), en un distillat consistant en solvant contenant de l'aniline et un résidu de distillation consistant essentiellement en le produit recherché et
h) on partage le distillat obtenu à l'opération de distillation (11), éventuellement après addition d'aniline fraîche, en deux courants partiels et on utilise un courant partiel au début des opérations (3) ou (5) et l'autre courant partiel, avec la phase organique quittant l'opération d'extraction supplémentaire (9), en tant qu'agent d'extraction pour la phase aqueuse à l'opération d'extraction du produit (8).

2. Procédé selon la revendication 1, caractérisé en ce que la phase aqueuse quittant l'opération d'extraction du produit (8), avant réutilisation, est débarrassée par distillation dans un évaporateur d'eau (10) d'une quantité d'eau représentant jusqu'à 80% du poids de l'eau qu'elle contient et, le cas échéant après passage dans un stade lavage (12) dans lequel on élimine les résidus de catalyseur de la phase organique provenant de (8), recyclée à un endroit appoprié après le dernier stade de réaction (6) et avant le stade d'extraction du produit (8).

3. Procédé selon les revendications 1 et 2, caractérisé en ce que la phase aqueuse contenant le catalyseur et qu'on doit réutiliser est séparée avant sa réutilisation en deux courants partiels, un courant partiel est renvoyé au premier stade de réaction (5) et l'autre est renvoyé dans le mélange de réaction après le premier stade de réaction et avant le dernier stade de réaction (6).
